# EUROPÄISCHE PATENTANMELDUNG

(11) **EP 1 712 257 A1**
(43) Veröffentlichungstag der Anmeldung: **18.10.2006**
(21) Anmeldenummer: 06110043.4
(22) Anmeldetag: 16.02.2006
(51) Int. Cl.: A61Q 5/08, A61K 8/23, A61K 8/37, A61K 8/25, A61K 8/73

(54) **Pastenförmige Blondiermittel mit cyclischen Kohlensäureestern und Silikaten**

(30) Priorität: 13.04.2005 DE 102005017196
(71) Anmelder: Henkel Kommanditgesellschaft auf Aktien, 40589 Düsseldorf (DE)
(72) Erfinder: Höffkes, Horst, 40595, Düsseldorf (DE); Brockmann, Claudia, 40627, Düsseldorf (DE)

(57) **Zusammenfassung**

Die Erfindung betrifft pastenförmige Mittel zum Blondieren menschlicher Haare auf Basis mindestens einer festen Peroxoverbindung und mindestens eines cyclischen Kohlensäureesters sowie mindestens eines Silikats.

## Beschreibung

Die Erfindung betrifft ein Blondiermittel für menschliche Haare, das in Pastenform vorliegt.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepaßt werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die sogenannten Blondierverfahren, im wesentlichen nur dunkelblonde oder hellere Haare. Die Grundlagen der Blondierverfahren sind dem Fachmann bekannt und können in einschlägigen Monographien, z.B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

So werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung mit einer Wasserstoffperoxidlösung vermischt werden, werden im weiteren als "Blondiermittel" bzw. "Blondierpasten" bezeichnet. Alle aufgeführten Mengenangaben beziehen sich, soweit nicht anders ausgeführt, ausschließlich auf diese Zubereitungen.

Weder die pastenförmigen noch die pulverförmigen Blondiermittel, die heute auf dem Markt sind, können als optimal angesehen werden. Während die Blondierwirkung auf dem Haar als befriedigend bezeichnet werden kann, bestehen doch noch eine Reihe von Nachteilen und Problemen sowohl bei Herstellung als auch bei der Handhabung dieser Mittel. Bei pastenförmigen Mitteln, die aus Stabilitätsgründen hochviskos eingestellt werden, können insbesondere die Sedimentationsstabilität und das Mischungsverhalten in der Wasserstoffperoxidlösung noch nicht befriedigen. Darüber hinaus sind einige der in Blondierpasten eingesetzten Strukturgeber substantiv zu Haar, so daß sie nach der kosmetischen Anwendung nur schwer vom Haar zu entfernen sind. Dies führt zumeist zu einer unangenehmen "Beschwerung des Haares, die u.a. einen schlechteren Halt der Frisur bewirkt.

Es wurde nun überraschenderweise gefunden, daß Blondierpasten mit verbessertem Sedimentationsverhalten, sehr guter Lagerfähigkeit und weiteren vorteilhaften Eigenschaften erhalten werden, wenn diese Pasten bestimmte Substanzen aus der Gruppe der Kohlensäureester und Silikate enthalten. Dies ist insbesondere daher überraschend, weil viele dieser Ester, und sogar erfindungsgemäß bevorzugte, selbst als relativ instabil gegenüber Oxidationsmitteln gelten.

Gegenstand der vorliegenden Erfindung sind daher pastenförmige Mittel zum Blondieren keratinischer Fasern, insbesondere menschlicher Haare, enthaltend
a) mindestens eine feste Peroxoverbindung,
b) mindestens einen cyclischen Kohlensäureester,
c) mindestens ein Silikat.

Die Bezeichnung "Paste" ist ein nicht scharf definierter Begriff für Festkörperdispersionen in Flüssigkeiten von teigiger Konsistenz, der im Rahmen der vorliegenden Erfindung weit auszulegen ist.

Die erfindungsgemäßen Blondiermittel enthalten als erste zwingende Komponente eine feste Peroxoverbindung. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten.

Bevorzugte erfindungsgemäße Mittel sind daher dadurch gekennzeichnet, daß die feste Peroxoverbindung ausgewählt ist aus Ammonium- und Alkalimetall-persulfaten und - peroxidisulfaten, wobei besonders bevorzugte erfindungsgemäße Mittel mindestens 2 verschiedene Peroxidisulfate enthalten.

Die Peroxoverbindungen sind in den erfindungsgemäßen Blondiermitteln bevorzugt in Mengen von 1 bis 80 Gew.%, vorzugsweise von 5 bis 70 Gew.%, besonders bevorzugt von 20 bis 65 Gew.% und insbesondere von 35 bis 60 Gew.%, jeweils bezogen auf das gesamte Mittel, enthalten.

Als weitere Komponente können die erfindungsgemäßen Blondiermittel ein Alkalisierungsmittel enthalten, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxyde, -carbonate, -hydrogencarbonate, -hydroxycarbonate, - silikate, insbesondere -metasilikate, sowie Alkaliphosphate verwendet werden. In einer bevorzugten Ausführungsform enthalten die erfindungsgemäßen Blondierpulver mindestens zwei unterschiedliche Alkalisierungsmittel. Dabei können Mischungen beispielsweise aus einem Metasilikat und einem Hydroxycarbonat bevorzugt sein.

Die erfindungsgemäßen Blondiermittel enthalten Alkalisierungsmittel bevorzugt in Mengen von 5 - 30 Gew.-%, insbesondere von 15 - 25 Gew.-%.

Als weitere zwingende Komponente enthalten die erfindungsgemäßen Blondiermittel mindestens einen cyclischen Kohlensäureester. Diese Kohlensäureester sind ringförmige 1,3-Dioxo-2-on-Verbindungen, die sich beispielsweise durch Reaktion von Phosgen mit Diolen erhalten lassen. Besonders bevorzugte cyclische Kohlensäureester weisen Ringgrößen von 5 oder 6 Atomen auf, sind demnach Dioxolane bzw. Dioxane.

Die erfindungsgemäß eingesetzten cyclischen Kohlensäureester weisen vorzugsweise niedrige Schmelz- und hohe Siedepunkte auf. Bevorzugte erfindungsgemäße Mittel sind dadurch gekennzeichnet, daß sie mindestens einen bei 60°C flüssigen cyclischen Kohlensäureester enthalten, wobei bevorzugte Kohlensäureester Siedepunkte von 100 °C oder darüber aufweisen.

Unabhängig von der Struktur der cyclischen Kohlensäureester werden diese in den erfindungsgemäßen Mitteln vorzugsweise in bestimmten Mengen eingesetzt. Hier sind erfindungsgemäße Mittel bevorzugt, die den bzw. die Kohlensäureester in Mengen von 1 bis 80 Gew.%, vorzugsweise von 5 bis 70 Gew.%, besonders bevorzugt von 10 bis 60 Gew.% und insbesondere von 25 bis 50 Gew.%, jeweils bezogen auf das gesamte Mittel, enthalten.

Mit Vorzug werden erfindungsgemäße cyclische Kohlensäureester eingesetzt, die einen Ring aus fünf Atomen aufweisen. Diese cyclischen Ester der Kohlensäure werden als 1,3-Dioxolan-2-one bezeichnet und lassen sich durch folgende Struktur beschreiben:

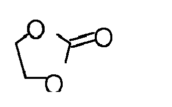

Im Grundkörper der 1,3-Dioxolan-2-one sind in 4 und 5-Stellung jeweils zwei H-Atome gebunden. Es ist im Rahmen der vorliegenden Erfindung möglich und bevorzugt, auch Derivate dieser Grundstruktur, also in 4- oder 4- und 5-Stellung substituierte 1,3-Dioxolan-2-one, einzusetzen. Hierbei sind der strukturellen Vielfalt keine Grenzen gesetzt, so daß sich mono-, di-, tri- und tetra-substituierte 1,3-Dioxolan-2-one zum Einsatz im Rahmen der vorliegenden Erfindung eignen.

Erfindungsgemäß bevorzugte Mittel sind daher dadurch gekennzeichnet, daß sie mindestens einen Kohlensäureester aus der Gruppe der substituierten oder unsubstituierten 1,3-Dioxolan-2-one 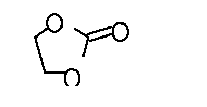 enthalten.

Besonders bevorzugt sind neben dem unsubstituierten 1,3-Dioxolan-2-on insbesondere die in 4-Stellung monosubstituierten Derivate, der nachstehenden Formel 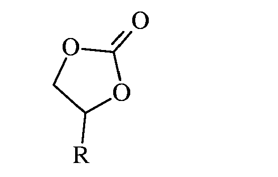 in der R für einen substituierten oder unsubstituierten Alkyl-, Alkenyl- oder Alkylarylrest steht. Bevorzugte Reste R sind Methyl-, Ethyl-, n-Propyl-, iso-Propyl- sowie Hydroxymethyl- und Hydroxyethyl-Reste.

Besonders bevorzugte erfindungsgemäße Mittel sind daher dadurch gekennzeichnet, daß sie mindestens ein in 4 Stellung monosubstituiertes 1,3-Dioxolan-2-on-Derivat der nachstehenden Formel (I) 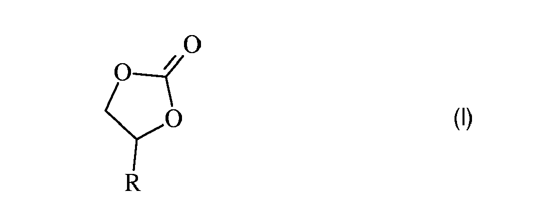 enthalten, in der R für einen substituierten oder unsubstituierten Alkyl-, Alkenyl- oder Alkylarylrest steht.

Noch weiter bevorzugte erfindungsgemäße Mittel dieser Ausführungsform sind dadurch gekennzeichnet, daß der Rest R in Formel (I) ausgewählt ist aus Methyl-, Ethyl-, n-Propyl-, iso-Propyl- sowie Hydroxymethyl- und Hydroxyethyl-Resten.

Besonders bevorzugte 1,3-Dioxolan-2-one stammen aus der Gruppe Ethylencarbonat (R = H), Propylencarbonat (R = CH₃) und Glycerincarbonat (R = CH₂OH).

Ethylencarbonat ist eine farblose kristalline Verbindung, die bei 39°C schmilzt und bei 238°C siedet. Das in Wasser, Alkoholen und organischen Lösungsmitteln leicht lösliche Ethylencarbonat ist über großtechnische Synthesen aus Ethylenoxid und flüssigem CO₂ herstellbar. Propylencarbonat ist eine wasserhelle, leichtbewegliche Flüssigkeit, mit einer Dichte von 1,2057 gcm⁻³, der Schmelzpunkt liegt bei -49°C, der Siedepunkt bei 242°C. Auch Propylencarbonat ist großtechnisch durch Reaktion von Propylenoxid und CO₂ bei 200°C und 80 bar zugänglich. Glycerincarbonat ist durch Umesterung von Ethylencarbonat oder Dimethylcarbonat mit Glycerin zugänglich, wobei als Nebenprodukte Ethylenglycol bzw. Methanol anfallen. Ein weiterer Syntheseweg geht von Glycidol (2,3-Epoxy-1-propanol) aus, das unter Druck in Gegenwart von Katalysatoren mit CO₂ zu Glycerincarbonat umgesetzt wird. Glycerincarbonat ist eine klare, leichtbewegliche Flüssigkeit mit einer Dichte von 1,398 gcm⁻³, die bei 125-130°C (0,15 mbar) siedet.

Besonders bevorzugt ist im Rahmen der vorliegenden Erfindung die Verwendung von Glycerincarbonat, das beispielsweise in Mengen von 1,0 bis 15,0 Gew.-%, vorzugsweise von 2,0 bis 13,5 Gew.-% und insbesondere von 3,5 bis 11,5 Gew.-%, jeweils bezogen auf das Mittel, eingesetzt wird.

Zusätzlich zu den Fünfring-Kohlensäureestern oder an ihrer Stelle können auch Sechsring-Kohlensäureester in den erfindungsgemäßen Mitteln enthalten sein. Bei dieser Ausführungsform der vorliegenden Erfindung werden mit Vorzug erfindungsgemäße cyclische Kohlensäureester eingesetzt, die einen Ring aus sechs Atomen aufweisen. Diese cyclischen Ester der Kohlensäure werden als 1,3-Dioxan-2-one bezeichnet und lassen sich durch ein Sechsringstruktur beschreiben. Sie können auch als Kohlensäureester von - gegebenenfalls substituiertem - Trimethylolpropan aufgefaßt werden.

Erfindungsgemäß bevorzugte Mittel sind dadurch gekennzeichnet, daß sie mindestens einen Kohlensäureester aus der Gruppe der substituierten oder unsubstituierten 1,3-Dioxan-2-one enthalten.

Vorzugsweise werden substituierte 1,3-Dioxan-2-one eingesetzt, wobei in 5-Stellung substituierte Vertreter weiter bevorzugt sind. Erfindungsgemäß besonders bevorzugte Mittel sind dadurch gekennzeichnet, daß sie mindestens ein in 5-Stellung mono- oder disubstituiertes 1,3-Dioxan-2-on-Derivat enthalten, wobei 5-Hydroxymethyl-1,3-Dioxan-2-on, 5-Hydroxymethyl-5-Methyl-1,3-Dioxan-2-on und/oder 5-Hydroxymethyl-5-Ethyl-1,3-Dioxan-2-on bevorzugt sind.

Die erfindungsgemäßen Mittel enthalten als dritte wesentliche Komponente ein oder mehrere Silikate. Silikate sind Salze oder Ester der Orthokieselsäure Si(OH)₄ und deren Kondensationsprodukte. Vorzugsweise werden als Silikate im Rahmen der vorliegenden Erfindung die Salze der Orthokieselsäuren und darunter insbesondere deren Kondensationsprodukte, eingesetzt.

Obwohl die Silikate sehr unterschiedliche Strukturen haben können, liegt ihnen das folgende einfache Bauprinzip zugrunde: Jedes Si-Atom ist stets von 4 O-Atomen umgeben, und nur durch Eckenverknüpfung dieser SiO₄-Einheiten entstehen die einzelnen Silikatklassen, bei denen man 6 Haupttypen unterscheidet:
1. Silikate mit selbständigen, "diskreten" Anionen:
   a) *Nesosilicate (Inselsilicate):* Dies sind *Orthosilicate* mit dem Anion [SiO₄]⁴⁻;
   b) *Sorosilicate (Gruppensilicate):* Hier sind die [SiO₄]-Tetraeder zu einer endlichen Gruppe verknüpft; dazu gehören u.a. die Di-Silikate mit dem Anion [Si₂O₇]^{6 -} und eine Anzahl z.T. synthetisch hergestellter Tri-Silikate;
   c) *Cyclosilicate (Ringsilicate):* In diesen Silikaten sind die [SiO₄]-Tetraeder zu Ringen angeordnet;
*2. lnosilicate (Kettensilicate u. Bandsilicate):* In diesen Silikaten sind die [SiO₄]-Tetraeder zu Ketten zusammengelagert, d.h. zu eindimensional unbegrenzten Gebilden, die praktisch Polymere des Anions [SiO₃]²⁻ sind.
*3. Phyllosilicate (Blattsilicate, Schichtsilicate):* In diesen Silikaten sind die [SiO₄]-Tetraeder jeweils in einer Ebene miteinander verkettet; sie bilden also Schichtengitter (Silikate mit doppelt gekoppelten Anionen).
*4. Tectosilicate (Gerüstsilicate):* In diesen Silikaten setzt sich die Verkettung der [SiO₄]-Tetraeder in allen drei Raumrichtungen fort (dreidimensionale Netzwerke).

Unabhängig davon, welche(s) Silikat(e) erfindungsgemäß eingesetzt wird/werden, sind erfindungsgemäße Mittel bevorzugt, die das bzw. die Silikat(e) in Mengen von 0,01 bis 10 Gew.%, vorzugsweise von 0,05 bis 5 Gew.%, besonders bevorzugt von 0,1 bis 2,5 Gew.% und insbesondere von 0,25 bis 1,5 Gew.%, jeweils bezogen auf das gesamte Mittel, enthalten.

Mit besonderem Vorzug werden erfindungsgemäß als Silikate Kieselsäuren eingesetzt. Hier sind erfindungsgemäße Mittel bevorzugt, die als Silikat Kieselsäure(n), vorzugsweise pyrogene Kieselsäure(n), enthalten.

Der Begriff "Kieselsäuren" ist dabei die Sammelbezeichnung für Verbindungen der allgemeinen Formel (SiO₂)ₘ × nH₂O. Setzt man ein Siliciumhalogenid (z.B. SiCl₄) mit Wasser um, so bildet sich primär die *Orthokieselsäure* (Monokieselsäure):

SiCl₄ + 4 H₂O → Si(OH)₄ + 4 HCl

Als erstes Kondensationsprodukt tritt die *Dikieselsäure* (Pyrokieselsäure, H₆Si₂O₇) auf:

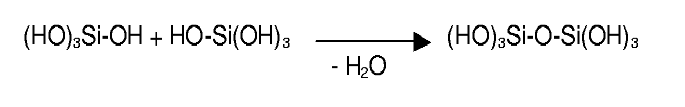

Weitere Kondensation führt auf dem Weg über cyclische Kieselsäuren (insbesondere [Si(OH)₂- O - ]₄) und käfigartige Kieselsäuren zu annähernd kugelförmigen *Polykieselsäuren.*

Formales Endprodukt der Kondensation ist polymeres Siliciumdioxid, (SiO₂)ₓ, das Anhydrid der Kieselsäure. Bei der Kondensation laufen kettenverlängernde, ringbildende und verzweigende Prozesse nebeneinander ab, so daß die Polykieselsäuren ungeordnet aufgebaut (amorph) sind. Die Si-Atome befinden sich bei allen Kieselsäuren im Mittelpunkt von unregelmäßig miteinander verknüpften Tetraedern, an deren 4 Eckpunkten O-Atome liegen, die gleichzeitig den Nachbartetraedern angehören.

Hinsichtlich des Produktionsumfanges haben die *Fällungskieselsäuren* die bei weitem größte Bedeutung. Sie werden aus einer wäßrigen Alkalisilicat-Lösung durch Fällung mit Mineralsäuren hergestellt. Dabei bilden sich kolloidale Primärteilchen, die mit fortschreitender Reaktion agglomerieren und schließlich zu Aggregaten verwachsen.

Unter der Bezeichnung *pyrogene Kieselsäuren* werden hochdisperse Kieselsäuren zusammengefaßt, die durch Flammenhydrolyse hergestellt werden. Dabei wird Siliciumtetrachlorid in einer Knallgas-Flamme zersetzt:

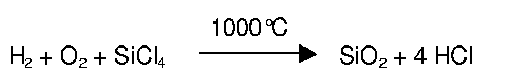

Pyrogene Kieselsäuren besitzen an ihrer nahezu porenfreien Oberfläche deutlich weniger OH-Gruppen als Fällungs- Kieselsäuren. Wegen ihrer durch die Silanol-Gruppen bedingten Hydrophilie werden die synthetischen Kieselsäuren häufig chemischen Nachbehandlungsverfahren unterzogen, bei denen die OH-Gruppen z.B. mit organischen Chlorsilanen reagieren. Dadurch entstehen modifizierte, z.B. hydrophobe Oberflächen, welche die anwendungstechnischen Eigenschaften der Kieselsäuren wesentlich erweitern.

Im Rahmen der vorliegenden Erfindung als Silikat besonders bevorzugte Kieselsäuren weisen spezifische Oberflächen (nach DIN 66131: 1993-07) von 50 bis 600 m²/g auf. Weiter bevorzugte im Rahmen der vorliegenden Erfindung als Silikat besonders bevorzugte Kieselsäuren besitzen Ölzahlen (nach DIN EN ISO 787-5: 1995-10) zwischen 30 und 300 g/100g sowie Primärpartikelgrößen zwischen 5 und 50 nm.

Um zu pastenförmigen Blondiermitteln zu gelangen, können die Inhaltsstoffe a) bis c) der erfindungsgemäßen Mittel - sofern sie in Partikelform vorliegen - mit den anderen Inhaltstoffen - die in flüssiger Form vorliegen können - vermischt werden. Gegebenenfalls ist eine inerte Matrix vorteilhaft, in der die Feststoffe suspendiert und die Flüssigkomponenten emulgiert oder gelöst werden.

Weiterhin hat es sich als vorteilhaft erwiesen, wenn die erfindungsgemäßen Blondiermittel nichtionogene grenzflächenaktive Stoffe enthalten. Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Besonders bervorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, daß die gewählten Substanzen bevorzugt frei von Lösungsmitteln, Stellmitteln und anderen Begleitstoffen sein sollen.

Bevorzugte nichtionogene grenzflächenaktive Stoffe sind
- alkoxylierte Fettalkohole mit 8 bis 22, insbesondere 10 bis 16, Kohlenstoffatomen in der Fettalkylgruppe und 1 bis 30, insbesondere 1 bis 15 Ethylenoxid- und/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal® B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C1-C4-Alkyl-Ethers, "verschlossen". Ein Beispiel für eine solche Verbindung ist das Handelsprodukt Dehypon® O 054, ein C₁₂₋₁₈-Fettalkoholol + 4,5 Ethylenoxid-butylether.
- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure.
- alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.
- Polyglycerinester und alkoxylierte Polyglycerinester. Bevorzugte Verbindungen dieser Klasse sind beispielsweise Poly(3)glycerindüsostearat (Handelsprodukt: Lameform® TGI (Henkel)) und Poly(2)glycerinpolyhydroxystearat (Handelsprodukt: Dehymuls® PGPH (Henkel)).
- Sorbitan-Fettsäureester und alkoxylierte Sorbitan-Fettsäureester wie beispielsweise Sorbitanmonolaurat und Sorbitanmonolaurat + 20 Ethylenoxid (EO).
- Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten. Bevorzugte Vertreter dieser Klasse sind beipielsweise Nonylphenol + 4 EO, Nonylphenol + 9 EO, Octylphenol + 3 EO und Octylphenol + 8 EO.

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich erfindungsgemäße Mittel erwiesen, die nichtionogene grenzflächenaktive Substanzen in Mengen von 1 - 5 Gew.-% enthalten.

Weiterhin können die erfindungsgemäßen Blondiermittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.

Bevorzugte anionische Tenside sind Alkylsulfate, Ethercarbonsäuresalze mit 10 bis 18 C-Atomen in der Alkylgruppe und bis zu 12 Glykolethergruppen im Molekül wie C₁₂H₂₅₋(C₂H₄O)₆-CH₂-COONa sowie insbesondere Salze von gesättigten und speziell ungesättigten C8-C22-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-.%, vor.

Als nichtionische Tenside eignen sich insbesondere C8-C22-Alkylmono- und oligoglycoside und deren ethoxylierte Analoga. Insbesondere die nichtethoxylierten Verbindungen, die zudem in Pulverform kommerziell erhältlich sind, haben sich als besonders geeignet erwiesen.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid® S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie das unter der Bezeichnung Dehyquart® F 75 in Abmischung mit Cetearylalkohle erhältliche Distearoylethylhydroxyethylammoniummethosulfat.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether und andere, als Feststoff stabile bzw. im Handel erhältliche Verbindungen,
- zwitterionische und amphotere Polymere, die als Feststoffe stabil bzw. bevorzugt als Handelsprodukte erhältlich sind,
- anionische Polymere wie beispielsweise Polyacrylsäuren, vernetzte Polyacrylsäuren und Vinylacetat/Crotonsäure-Copolymere, sofern diese als Feststoffe stabil bzw. bevorzugt im Handel erhältlich sind,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Farbstoffe zum Einfärben der Zubereitungen,
- Wirkstoffe wie Panthenol, Pantothensäure, Allantoin, Pyrrolidoncarbonsäuren und deren Salze,
- Cholesterin,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Carbonate, Hydrogencarbonate, Guanidine, Harnstoffe sowie primäre, sekundäre und tertiäre Phosphate,

Die Auswahl dieser weiteren Stoffe wird der Fachmann gemäß der gewünschten Eigenschaften der Mittel treffen.

Die erfindungsgemäßen Mittel können darüber hinaus weitere Inhaltsstoffe enthalten. So hat sich z.B. der Einsatz von sogenannten Ölkomponenten als vorteilhaft erwiesen.

Mit besonderem Vorzug enthalten die erfindungsgemäßen Mittel zusätzlich eine Ölkomponente (D). Unter Ölkomponenten sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als Fettsäuren (D1) können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 -10 Gew.%, wobei ganz besonders vorteilhaft Mengen von 1 - 5 Gew.% sein können.

Als Fettalkohole (D2) können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rndertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol® , z.B. Stenol® 1618 oder Lanette® , z.B. Lanette® O oder Lorol® , z.B. Lorol® C8, Lorol® C14, Lorol® C18, Lorol® C8-18, HD-Ocenol® , Crodacol® , z.B. Crodacol® CS, Novol® , Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona® , White Swan® , Coronet® oder Fluilan® käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 -20 Gew.-% eingesetzt.

Als natürliche oder synthetische Wachse (D3) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Insbesondere bevorzugt sind sogenannte kosmetische Ölkörper als Inhaltsstoffe der erfindungsgemäßen Mittel.
Zu den natürlichen und synthetischen kosmetischen Ölkörpern (D4) sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyln-undecylether, n-Undecyl-n-dodecylether und n-Hexyl-n-Undecylether sowie Di-tert-butylether, Di-iso-pentylether, Di-3-ethyldecylether, tert.-Butyl-n-octylether, isoPentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol® ), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I),

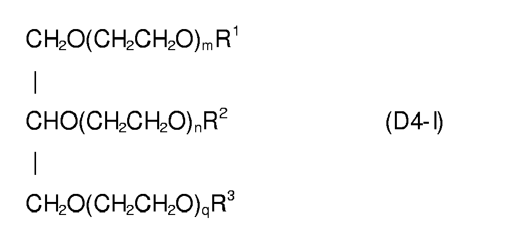

in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, daß mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Zusammenfassend sind insbesondere erfindungsgemäße Mittel bevorzugt, die zusätzlich eine inerte Ölkomponente, vorzugsweise aus der Gruppe der Ester langkettiger Fettsäuren, besonders bevorzugt aus der Gruppe der Alkyloleate und/oder Alkylstearate, enthalten.

Für die Anwendung der erfindungsgemäßen Mittel auf dem Haar werden die pastenförmigen Blondiermittel unmittelbar vor dem Auftragen mit einer Wasserstoffperoxid-Lösung vermischt. Die Konzentration dieser Wasserstoffperoxid-Lösung wird einerseits von den gesetzlichen Vorgaben und andererseits von dem gewünschten Effekt bestimmt; in der Regel werden 6- bis 12-prozentige Lösungen in Wasser oder wasserstoffperoxidhaltige Emulsionen verwendet.

Das Mischungsverhältnis von Blondiermittelsuspension zu Oxidationsmittelzubereitung beträgt vorzugsweise 2:1 bis 1:8, insbesondere 1:1 bis 1:5. Die Mengenverhältnisse von Blondierpaste und Wasserstoffperoxid-Lösung liegen dabei üblicherweise im engeren Bereich 1:1 bis 1:3, wobei ein Überschuß an Wasserstoffperoxid-Lösung insbesondere dann gewählt wird, wenn keine zu ausgeprägte Blondierwirkung erwünscht ist.

Das nach dem Vermischen mit der Oxidationsmittelzubereitung erhaltene gebrauchsfertige Mittel zum Entfärben oder Blondieren von Haaren weist einen pH-Wert von etwa 7,5 bis 11, insbesondere von 8 bis 10,0, auf.

Das gebrauchsfertige Mittel wird auf das Haar gleichmäßig aufgetragen und nach einer Einwirkungszeit von 15 bis 60 Minuten bei Raumtemperatur (20-25°C) beziehungsweise von 10 bis 50 Minuten bei Wärmeeinwirkung (30-50°C) mit Wasser ausgespült.

Die Blondiermittelpasten der vorliegenden Erfindung können je nach ihrer Viskosität in Tuben, Sachets oder Tiegel abgefüllt werden. Neben der anwendungsfreundlichen Produktviskosität über einen großen Temperaturbereich und der leichten Anmischbarkeit mit dem Oxidationsmittel zeichnet sich das erfindungsgemäße Mittel durch eine hervorragende Lagerstabilität, Auftragefähigkeit, Verteilbarkeit und Haftung am Haar sowie ein breites Anwendungsspektrum aus. Im Vergleich zu herkömmlichen Blondiermitteln läßt sich das erfindungsgemäße Blondiermittel sehr leicht mit Wasser wieder rückstandsfrei aus dem Haar ausspülen.

Ein weiterer Gegenstand der vorliegenden Erfindung ist die Verwendung von Mischungen aus cyclischen Kohlensäureestern und Silikaten in Mitteln zum Blondieren menschlicher Haare.

Bezüglich bevorzugter erfindungsgemäßer Verwendungen gilt mutatis mutandis das zu den bevorzugten Mitteln Ausgeführte.

Die nachfolgenden Beispiele sollen den Gegenstand näher erläutern.

### Beispiele:

Es wurden folgende Mischungen hergestellt [Einwaage in g]:

| Rezeptur | E1 | E2 | V1 |
|---|---|---|---|
| Ammoniumperoxidisulfat | 62,5 | 62,5 | 62,5 |
| Aerosil® 200* | 0,5 | 0,5 | 0,5 |
| Glycerincarbonat | 45,0 | 65,0 | - |
| Elfan® AT 84** | 5,0 | 5,0 | 5,0 |
| EDTA | 2,5 | 2,5 | 2,5 |
| Trinatriumphosphat | 4,0 | 4,0 | 4,0 |
| Cekol 50.000*** | 7,5 | 7,5 | 7,5 |
| Isooctylstearat | - | - | 45,0 |

| | | | |
|---|---|---|---|
| * pyrogene Kieselsäure ** INCI-Bezeichnung: Cocoyl Isethionate *** INCI-Bezeichnung : Cellulose Gum | | | |

Die physikalischen Eigenschaften wurden direkt nach der Herstellung und nach 3 Wochen Lagerung beurteilt:
- E1:: mittelviskose, homogene Paste; auch nach 3 Wochen keine Sedimentation
- E2:: weiche, homogene Paste; auch nach 3 Wochen keine Sedimentation
- V1:: weiche Paste; bereits nach einem Tag starke Ölabscheidung; nach 3 Wochen vollständig sedimentiert

Die Ergebnisse zeigen, daß nur die erfindungsgemäßen Zusammensetzungen zufriedenstellende Viskositäten und Stabilitäten aufweisen, während Zusammensetzungen ohne Kohlensäureester nicht ausreichend stabil sind.

## Patentansprüche

1. Pastenförmiges Mittel zum Blondieren keratinischer Fasern, insbesondere menschlicher Haare, enthaltend
a) mindestens eine feste Peroxoverbindung,
b) mindestens einen cyclischen Kohlensäureester,
c) mindestens ein Silikat.

2. Mittel nach Anspruch 1, **dadurch gekennzeichnet, daß** es mindestens einen bei 60°C flüssigen cyclischen Kohlensäureester enthält, wobei bevorzugte Kohlensäureester Siedepunkte von 100°C oder darüber aufweisen.

3. Mittel nach einem der Ansprüche 1 oder 2, **dadurch gekennzeichnet, daß** es den bzw. die Kohlensäureester in Mengen von 1 bis 80 Gew.%, vorzugsweise von 5 bis 70 Gew.%, besonders bevorzugt von 10 bis 60 Gew.% und insbesondere von 25 bis 50 Gew.%, jeweils bezogen auf das gesamte Mittel, enthält.

4. Mittel nach einem der Ansprüche 1 bis 3, **dadurch gekennzeichnet, daß** es mindestens einen Kohlensäureester aus der Gruppe der substituierten oder unsubstituierten 1,3-Dioxolan-2-one 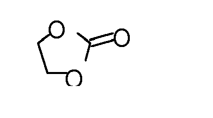 enthält.

5. Mittel nach Anspruch 4, **dadurch gekennzeichnet, daß** es mindestens ein in 4 Stellung monosubstituiertes 1,3-Dioxolan-2-on-Derivat der nachstehenden Formel (I) 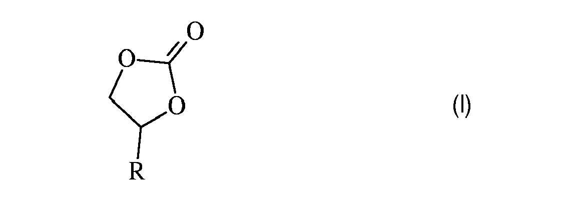 enthält, in der R für einen substituierten oder unsubstituierten Alkyl-, Alkenyl- oder Alkylarylrest steht.

6. Mittel nach Anspruch 5, **dadurch gekennzeichnet, daß** der Rest R in Formel (I) ausgewählt ist aus Methyl-, Ethyl-, n-Propyl-, iso-Propyl- sowie Hydroxymethyl- und Hydroxyethyl-Resten.

7. Mittel nach einem der Ansprüche 1 bis 6, **dadurch gekennzeichnet, daß** es mindestens einen Kohlensäureester aus der Gruppe der substituierten oder unsubstituierten 1,3-Dioxan-2-one enthält.

8. Mittel nach Anspruch 7, **dadurch gekennzeichnet, daß** es mindestens ein in 5 Stellung mono- oder disubstituiertes 1,3-Dioxan-2-on-Derivat enthält, wobei 5-Hydroxymethyl-1,3-Dioxan2-on, 5-Hydroxymethyl-5-methyl-1,3-dioxan2-on und/oder 5-Hydroxymethyl-5-ethyl-1,3-dioxan2-on bevorzugt sind.

9. Mittel nach einem der Ansprüche 1 bis 8, **dadurch gekennzeichnet, daß** es die feste(n) Peroxoverbindung(en) in Mengen von 1 bis 80 Gew.%, vorzugsweise von 5 bis 70 Gew.%, besonders bevorzugt von 20 bis 65 Gew.% und insbesondere von 35 bis 60 Gew.%, jeweils bezogen auf das gesamte Mittel, enthält, wobei bevorzugte Peroxoverbindungen ausgewählt sind aus Ammonium- und Alkalimetall-persulfaten und -peroxidisulfaten.

10. Mittel nach einem der Ansprüche 1 bis 9, **dadurch gekennzeichnet, daß** es mindestens 2 verschiedene Peroxidisulfate enthält.

11. Mittel nach einem der Ansprüche 1 bis 10, **dadurch gekennzeichnet, daß** es das bzw. die Silikat(e) in Mengen von 0,01 bis 10 Gew.%, vorzugsweise von 0,05 bis 5 Gew.%, besonders bevorzugt von 0,1 bis 2,5 Gew.% und insbesondere von 0,25 bis 1,5 Gew.%, jeweils bezogen auf das gesamte Mittel, enthält.

12. Mittel nach einem der Ansprüche 1 bis 11, **dadurch gekennzeichnet, daß** es als Silikat Kieselsäure(n), vorzugsweise pyrogene Kieselsäure(n) enthält.

13. Mittel nach einem der Ansprüche 1 bis 12, **dadurch gekennzeichnet, daß** es zusätzlich eine inerte Ölkomponente, vorzugsweise aus der Gruppe der Ester langkettiger Fettsäuren, besonders bevorzugt aus der Gruppe der Alkyloleate und/oder Alkylstearate, enthält.

14. Verwendung von Mischungen aus cyclischen Kohlensäureestern und Silikaten in Mitteln zum Blondieren menschlicher Haare.
